# EUROPÄISCHE PATENTSCHRIFT

(11) **EP 1 966 125 B1**
(45) Veröffentlichungstag und Bekanntmachung des Hinweises auf die Patenterteilung: **27.06.2012**
(21) Anmeldenummer: 06830502.8
(22) Anmeldetag: 11.12.2006
(51) Int. Cl.: C07C 213/06, C07C 217/52

(54) **VERFAHREN ZUR HERSTELLUNG VON O-ALKYLIERTEN AMINOALKOHOLEN**
METHOD FOR PRODUCING O-ALKYLATED AMINOALCOHOLS
PROCEDE DE PRODUCTION D'AMINOALCOOLS O-ALKYLES

(30) Priorität: 22.12.2005 EP 05112826
(43) Veröffentlichungstag der Anmeldung: 10.09.2008
(73) Patentinhaber: BASF SE, 67056 Ludwigshafen (DE)
(72) Erfinder: SIEGEL, Wolfgang, 67117 Limburgerhof (DE); HADERLEIN, Gerd, 67269 Grünstadt (DE); STÄB, Tobias, 68165 Mannheim (DE)
(86) Internationale Anmeldenummer: PCT/EP2006/069528
(87) Internationale Veröffentlichungsnummer: WO 2007/074046

(56) Entgegenhaltungen:
- DE-A1- 10 344 447
- NISHI T ET AL: "STUDIES ON 2-OXOQUINOLINE DERIVATIVES AS BLOOD PLATELET AGGREGATION INHIBITORS. IV. SYNTHESIS AND BIOLOGICAL ACTIVITY OF THE METABOLITES OF 6-[4-(1-CYCLOHEXL-1H-5-TETRAZOLYL)BUTOXY]- 2-OXO-1,2,3,4-TETRAHYDRO QUINOLINE (OPC-13013)" CHEMICAL AND PHARMACEUTICAL BULLETIN, TOKYO, JP, Bd. 33, Nr. 3, 1985, Seiten 1140-1147, XP001247898 ISSN: 0009-2363 in der Anmeldung erwähnt

## Beschreibung

### Stand der Technik

Die vorliegende Erfindung ist auf ein Verfahren zur Herstellung von N-unsubstituierten und N-monosubstituierten Aminoalkylethern bzw. Aminobenzylethern gerichtet. Insbesondere beschäftigt sich die Erfindung mit der regioselektiven O-Alkylierung, bzw. O-Benzylierung von N-unsubstituierten und N-monosubstituierten Aminoalkoholen. Derartige Aminoalkylether bzw. Aminobenzylether sind wertvolle Intermediate zur Herstellung bioaktiver Wirkstoffe (EP 0691346; T. Nishi et al. Chem. Pharm. Bull. 1985, 33(3), 1140-1147; D. Lewis et al. Steroids, 1995, 60, 475-483; D. Kikelj et al. J. Med. Chem. 1998, 41, 530-539; M. G. N. Russell et al., J. Med. Chem. 1999, 42, 4981-5001; Fray et al. Bioorg. Med. Chem. Lett. 2001, 11, 567-570; Koert et al. Ang. Chem. Int. Ed. 2001, 40(11), 2076-2078; Price et al. Tetrahedron Letters 2004, 45, 5581-5583) sowie chirale Auxiliare für die chemische Synthese (T. K., Chakraborty, K, A. Hussain, G. V. Reddy, Tetrahedron 1995, 51(33), 9179-9190; A. Fadel, A. Khesrani, Tetrahedron: Asymmetry 1998, 9(2), 305-320; K. P. Chiev, S. Roland, P. Mageney, Tetrahedron: Asymmetry 2002, 13(20), 2205-2210; M. P. Bertrand, S. Coantic, L. Feray, R. Nouguier, P. Perfetti, Tetrahedron 2000, 56(24), 3951-3962; J. Lacour et al. J. Org. Chem. 2003, 68(16), 6304-6308; JP 59044345).

Etherbildungen gehören zu den Standardreaktionen der organischen Chemie, die auch im industriellen Maßstab durchgeführt werden (Organikum, VEB, Berlin 1986, S. 191ff.).

Prinzipiell unterscheidet man die saure und die basische Ethersynthese nach Williamson. Bei der Williamson-O-Alkylierung wird ein Alkoholatanion erzeugt und dieses mit einer Verbindung mit nukleofuger Abgangsgruppe, z.B. einem Alkylhalogenid, zur Reaktion gebracht.

Verbindungen mit nukleofuger Abgangsgruppe - also elektrophile Reagenzien - wie z.B. Alkylhalogenide, Alkylsulfate, -sulfonate, aber auch Benzylhalogenide oder dergleichen reagieren jedoch auch leicht mit nukleophilen Aminofunktionen eines organischen Moleküls. Will man jetzt einen Alkohol basisch verethern, der auch eine lungeschützte oder nur monosubtituierte Aminofunktion im Molekül enthält, so muss man Bedingungen finden, unter denen die Reaktion der Aminofunktion möglichst unterbleibt und die Alkoholfunktion vollständig umgesetzt wird. Derartige Synthesen werden im Schrifttum verschiedentlich und mit unterschiedlichem Erfolg aufgeführt. Zumeist handelt es sich dabei um die Reaktion des Aminoalkohols mit extrem starken, irreversibel reagierenden Basen, wobei zunächst das Alkalialkoholation gebildet wird und anschließend die Umsetzung mit dem Elektrophil erfolgt (Whitesell et al. J. Org. Chem. 1977, 42, 377; Mayer et al. J. Med. Pharm. Chem. 1961, 3, 409; Meyers et al. J. Org. Chem. 1978, 43, 892, Hu et al. Synth. Commun. 1995, 25(6), 907.). Im Falle des Einsatzes von Alkalihydriden muss zudem die gefährliche Wasserstoffentwicklung im Produktionsmaßstab unter Kontrolle gebracht werden.

In DE 103 44 447 A1 wird der Einsatz von Alkalialkoholaten als Deprotonierungsreagenz beschrieben, wobei die verwendeten Alkalialkoholate relativ kostenintensiv sind.

Aufgrund der Nachteile der Verfahren des Standes der Technik besteht daher weiterhin ein Bedarf an regioselektiv arbeitenden O-Alkylierungs- bzw. O-Benzylierungs-Synthesen von N-ungeschützten und N-monosubstituierten Aminoalkoholen, die vor allem im industriellen Maßstab vorteilhaft und kostengünstig einsetzbar sind.

### Aufgabenstellung

Die Aufgabe der vorliegenden Erfindung war deshalb die Angabe eines kostengünstigen Verfahrens zur regioselektiven O-Alkylierung, bzw. O-Benzylierung von N-ungeschützten und N-monosubtituierten Aminoalkoholen, welches im Gegensatz zum Stand der Technik auch in technischem Maßstab vorteilhaft anzuwenden ist. Insbesondere sollte das Verfahren vom ökonomischen und ökologischen Standpunkt aus gesehen den Verfahren des Standes der Technik überlegen sein und die Generierung der gewünschten Ether in verbesserten Ausbeuten und Regioselektivitaten bei geringeren Kosten erlauben.

### Gegenstand der Erfindung

Die Aufgabe wird anspruchsgemäß gelöst durch ein Verfahren zur Herstellung von O-alkylierten Aminoalkoholen durch Umsetzen von N-unsubstituierten oder N-monosubstituierten Aminoalkoholatsalzen mit Alkylhalogeniden, wobei die Aminoalkoholatsalze mittels Alkali- oder Erdalkalihydroxiden gebildet werden.

Die abhängigen Ansprüche betreffen bevorzugte Ausführungsformen des erfindurigsgemäßen Verfahrens.

Die erfindungsgemässe wird üblicherweise in einem Lösungsmittel durchgeführt. Der Fachmann orientiert sich bezüglich der Auswahl des Lösungsmittels an der Produktausbeute, Reaktionsgeschwindigkeit, Handhabbarkeit der entstehenden Alkoholat-Suspensionen und dem Preis des Lösungsmittels.

Von Vorteil sind Lösungsmittel, die sich mit dem Aminoalkohol mischen lassen, chemisch inert sind, d.h. nicht mit dem Aminoalkohol, einem Alkali- oder Erdalkalihydroxiden, dem enstehenden Wasser oder dem Alkylierungs- bzw. Benzylierungsmittel reagieren, und typischerweise einen Siedepunkt haben, der über dem des Wassers liegt, der aus dem Alkali- oder Erdalkalihydroxiden bei der Deprotonierung des Aminoalkohols entsteht.

Typische für die erfindungsgemässe Reaktion geeignete Lösungsmittel sind Aliphaten oder Aromaten mit entsprechenden Siedepunkten, auch Mischungen und Siedeschnitte.

Bevorzugt sind Aromaten, wie Toluol, ortho-Xylol, meta-Xylol, para-Xylol, Ethylbenzol, Methylethylbenzol, andere Alkylbenzole, etc. pp. oder Mischungen daraus: Besonders bevorzugt sind Xylol-Isomerengemische, da bei den eingesetzten Aminoalkoholen die gebildeten und ausgefällten Aminoalkoholatsalze in einer besonders gut handhabbaren und leicht vom Wasser zu befreienden Form anfallen. Zudem ist eine einfache Rezyklierung der Lösungsmittelströme möglich.

Die umzusetzenden N-unsubstituierten und N-monosubstituiertenAminoalkoholatsalze werden mittels Alkali- oder Erdalkalihydroxiden generiert. Die Alkali- oder Erdalkalihydroxiden können als Feststoff oder vorzugsweise in Wasser gelöst oder in einem inerten Verdünnungsmittel suspendiert in die Reaktion eingesetzt werden. In diesem Fall kann man zur Vervollständigung der Reaktion das sich bildende Wasser abdestillieren.

In einer besonderen Ausführungsform kann ein weiteres Lösungsmittel eingesetzt werden, dessen Siedepunkt zwischen dem sich bildendem Wasser und dem anderen Lösungsmittel liegt. Diese Reaktionsführung erlaubt die bessere Entfernung des Wassers. Als weiteres Lösungsmittel werden bevorzugt Alkohole, aliphatische und aromatische Ether und Ketone, sowohl cyclische als auch acylische, eingesetzt.

Besonders bevorzugt solche mit einer Kohlenstoffatomanzahl zwischen 2 und 10, insbesondere C2-C10-Alkohole, C2-C10-Ether und C2-C10-Ketone.

Prinzipiell ist das erfindungsgemässe Verfahren für eine Vielzahl von N-unsubstituierten und N-monosubstituierten Aminoalkoholen einsetzbar. Vorzugsweise setzt man als Aminoalkohole Verbindungen der allgemeinen Formel (I) oder (II) ein, 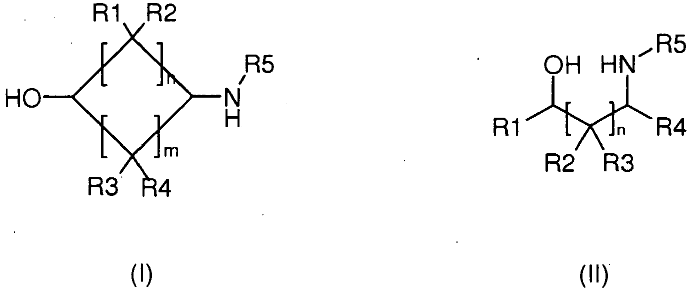 worin
unabhängig voneinander n = 0, 1, 2, 3, 4 und m = 1, 2, 3, 4 bedeuten und R1, R2, R3, R4 bzw. R5 unabhängig voneinander H, substituierte und unsubstituiert (C1-C8)-Alkyl, (C3-C8)-Cycloalkyl, (C1-C8)-Alkyl-(C3-C8)-Cycloalkyl, (C3-C8)-Cycloalkyl-((C1-C8)-Alkyl)1-3, (C2-C8)-Alkenyl, (C2-C8)-Alkinyl, (C6-C18)-Aryl, (C7-C19)-Aralkylrest, (C6-C18)-Aryl-((C1-C8)-Alkyl)1-3, (C3-C18)-Heteroarylrest, (C4-C19)-Heteroaralkyl, (C3-C18)-Heteroarylrest-((C1-C8)-Alkyl)1-3, bedeuten können.

R5 ist bevorzugt H oder ist im Falle von anderen Chiralitätszentren im Molekül über ein chirales Kohlenstoffatom an das Stickstoff-Atom gebunden.

Als (C1-C8)-Alkyl sind anzusehen: Methyl, Ethyl, n-Propyl, Isopropyl, n-Butyl, Isobutyl, sec-Butyl, tert.-Butyl, Pentyl, Hexyl, Heptyl oder Octyl einschließlich aller Bindungsisomeren.

Als (C2-C8)-Alkenyl ist mit Ausnahme von Methyl ein wie oben dargestellter (C1-C8)-Alkyl-Rest zu verstehen, der mindestens eine Doppelbindung aufweist.
Unter (C2-C8)-Alkinyl ist mit Ausnahme von Methyl ein wie oben dargestellter (C1-C8)-Alkyl-Rest zu verstehen, der mindestens eine Dreifachbindung aufweist.
Unter (C3-C8)-Cycloalkyl versteht man Cyclopropyl, Cyclobutyl, Cyclopentyl, Cyclohexyl bzw. Cycloheptylreste etc. Diese konnen N-, O-, atomhaltige Reste im Ring aufweise, wie z. B. 1-, 2-, 3-, 4-Piperidyl, 1-, 2-, 3-Pyrrolidinyl, 2-, 3-Tetrahydrofuryl, 2-, 3-, 4-morpholinyl.

Unter einem (C6-C18)-Arylrest wird ein aromatischer Rest mit 6 bis 18 C-Atomen verstanden. Insbesondere zahlen hierzu Verbindungen wie Phenyl-, Naphthyl-, Anthryl-, Phenanthryl-, Biphenylreste.

Ein (C7-C19)-Aralkylrest ist ein uber einen (C1-C8)-Alkylrest an das Molekul gebundener (C6-C18)-Arylrest.

Ein (C3-C18)-Heteroarylrest bezeichnet im Rahmen der Erfindung ein funf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Reste angesehen, wie 1-, 2-, 3-Furyl, wie 1-, 2-, 3-Pyrrolyl, 1-, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, , 2-, 4-, 5-, 6-Pyrimidinyl. Unter einem (C4-C19)-Heteroaralkyl wird ein dem (C7-C19)-Aralkylrest entsprechendes heteroaromatisches System verstanden.

Die oben definierten Reste können sowohl unsubstituiert als auch ein oder mehrfach substituiert sein durch Reste, die sich unter den Reaktionsbedingungen entweder inert verhalten oder die zuvor durch Schutzgruppen maskiert worden sind. Beispiele für Substituenten sind OH; NH₂, SH, NO₂, CN, CO, COOH, F, Cl, Br, J.

Unter dem Begriff enantiomerenangereichert wird im Rahmen der Erfindung der Anteil eines Enantiomers im Gemisch mit seiner optischen Antipode in einem Bereich von > 50% und < 100% verstanden.

Die eingesetzten N-unsubstituierten und N-monosubstituiertenen Aminoalkohole können achiral oder chiral sein. Sie können auch als racemische, enantiomerenangereicherte oder diasteremerenangereicherte Mischungen vorliegen. Bevorzugt ist der Einsatz von N-unsubstituieren oder N-monosubstituierten 2-Amino-cycloalkanolen oder, besonders bevorzugt von N-unsubstituieren oder N-monosubstituierten trans-2-Aminocycloalkanolen. Diese sind z.B. durch Ringöffnung der entsprechenden Epoxide mit Ammoniak bzw. monosubstituierten Aminen zugänglich.

Ganz besonders bevorzugt ist der Einsatz von N-unsubstituieren oder N-monosubstituierten trans-2-Aminocyclopentanol oder N-unsubstituieren oder N-monosubstituierten trans-2-Aminocyclohexanol.

Als Alkylhalogenide können alle dem Fachmann für diese Reaktion bekannten Verbindungen herangezogen werden. Vorzugsweise werden (C1-C8)-Alkylchloride oderbromide in die efindungsgemäße Reaktion eingesetzt. Ganz besonders bevorzugt sind hier primäre und sekundäre Alkylhalogenide, wovon solche mit Methyl- bzw. Ethylresten besonders zu empfehlen sind. Besonders bevorzugt sind Alkylchloride.

Als Benzylhalogenide können bevorzugt Benzylchlorid oder Benzylbromid verwendet werden, wobei die Verbindungen am Arylrest ein oder mehrfach durch gängige Substituenten substituiert sein können. Besonders bevorzugt ist Benzylchlorid.

Bei der erfindungsgemäßen Reaktion geht man vorzugsweise so vor, dass man im Lösungsmittel das Substrat und die Base bei Temperaturen von 20-200°C, vorzugsweise 100-150°C, besonders bevorzugt bei der Siedetemperatur des eingesetzten Lösungsmittels, vorlegt. Leichtsiedende Lösungsmittel wie insbesondere das entstehende Wasser und das eventuell eingesetzte zweite Lösungsmittel können anschließend durch Destillation entfernt werden. Danach gibt man das Alkylierungs- bzw. Benzylierungsmittel bei Temperaturen von 20-200°C, vorzugsweise 50-150°C, besonders bevorzugt bei der Siedetemperatur des eingesetzten Lösungsmittels zu. Der Druck, bei dem die Reaktion durchgeführt wird, ist an sich nicht kritisch. Aus praktischen Gründen wird die Reaktion bevorzugt bei 500-5000 hPa, besonders bevorzugt bei Normaldruck durchgeführt.

Nach vollendeter Reaktion kann man die Mischung ggf. abkühlen lassen und das ausgefallene anorganische Salz abfiltrieren oder auf eine andere dem Fachmann bekannte Art und Weise, beispielsweise mit Zentrifuge, Zyklotron etc., abtrennen. Alternativ kann das gebildete anorganische Salz auch in der Rohmischung verbleiben. Danach wird das Produkt beispielsweise durch Destillation, isoliert.

Die Destillation kann vorteilhaft durch einstufige Verdampfung, bevorzugt durch fraktionierende Destillation in einer oder mehreren, wie 2 oder 3 Destillationsapparaturen erfolgen. Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen, Füllkörperkolonnen, Kolonnen mit Seitenabzug oder Trennwandkolonnen.

Die Destillation kann in Batchfahrweise oder kontinuierlich durchgeführt werden. Wegen der Temperaturempfindlichkeit der Substrate wird die Destillation bevorzugt bei vermindertem Druck - in Abhängigkeit vom entsprechenden Reaktionsprodukt - von 1 bis 500 hPa, bevorzugt von 5 bis 200 hPa, durchgeführt.

Unter dem Begriff diastereomerenangereichert wird im Rahmen der Erfindung der Anteil eines Diastereomers im Gemisch mit anderen diastereomeren Isomeren in einem Bereich von > 50% und < 100% verstanden.

Die dargestellten chiralen Strukturen beziehen sich auf alle möglichen Diastereomere und Enantiomere (R-, S-) sowie deren Gemische und das Racemat.

### Auführungsbeispiele

### Beispiel 1

0,2 mol trans-2-Aminocyclohexanol werden in 600 ml Xylol-Isomerengemisch gelöst und auf Rückfluss erhitzt. Zu dieser Lösung wird über 15 Minuten 0,2 mol einer 50 %-igen Natrium- bzw. Kaliumhydroxid-Lösung in Wasser zugetropft. Dabei wird das eingetragene und das sich bildende Wasser sofort abdestilliert. Nach beendeter Zugabe destilliert man das in der Reaktionslösung vorhandene Wasser so lange weiter ab, bis eine Übergangtemperatur von 140-141 °C erreicht wird. Danach werden 0,2 mol Benzylchlorid über 30 Minuten zugetropft und die Lösung für zwei Stunden weiter unter Rückfluss gerührt. Nach wässriger Aufarbeitung erhält man im Falle von Natriumhydroxid das gewünschte 2-Benzyloxy-cyclohexylamin in 62 % (GC FI%) Ausbeute, im Fall von Kaliumhydroxid in 69 % (GC FI%) Ausbeute.

### Beispiel 2

0,2 mol trans-2-Aminocyclohexanol werden in 600 ml Xylol-Isomerengemisch und 100 ml n-Butanol gelöst und auf Rückfluss erhitzt. Zu dieser Lösung wird über 15 Minuten 0,2 mol einer 50 %-igen Natrium- bzw. Kaliumhydroxid-Lösung in Wasser zugetropft. Dabei wird das eingetragene und das sich bildende Wasser sofort abdestilliert. Nach beendeter Zugabe destilliert man das in der Reaktionslösung vorhandene Wasser, und danach das n-Butanol so lange ab, bis eine Übergangtemperatur von 140-141 °C erreicht wird. Danach werden 0,2 mol Benzylchlorid über 30 Minuten zugetropft und die Lösung für zwei Stunden weiter unter Rückfluss gerührt. Nach wässriger Aufarbeitung erhält man im Falle von Natriumhydroxid das gewünschte 2-Benzyloxy-cyclohexylamin in 93 % (GC FI%) Ausbeute, im Fall von Kaliumhydroxid in 92 % (GC FI%) Ausbeute.

## Patentansprüche

1. Verfahren zur Herstellung von O-alkylierten Aminoalkoholen bzw O-benzylierten Aminoalkoholen durch Umsetzen von N-unsubstituierten oder N-monosubstituierten Aminoalkoholatsalzen mit Alkylhalogeniden bzw. Benzylhalogeniden, wobei die Aminoalkoholatsalze mittels Alkali- oder Erdalkalihydroxiden gebildet werden.

2. Verfahren nach Anspruch 1, **dadurch gekennzeichnet, dass** die Reaktion in einem Lösungsmittel durchgeführt wird.

3. Verfahren nach Anspruch 1, **dadurch gekennzeichnet, dass** in einem organischen Lösungsmittel durchgeführt wird.

4. Verfahren nach Anspruch 1, **dadurch gekennzeichnet, dass** Xylol als Lösungsmittel verwendet wird.

5. Verfahren nach Anspruch 1, **dadurch gekennzeichnet, dass** der Aminoalkohol die Formeln (I) oder (II) besitzt 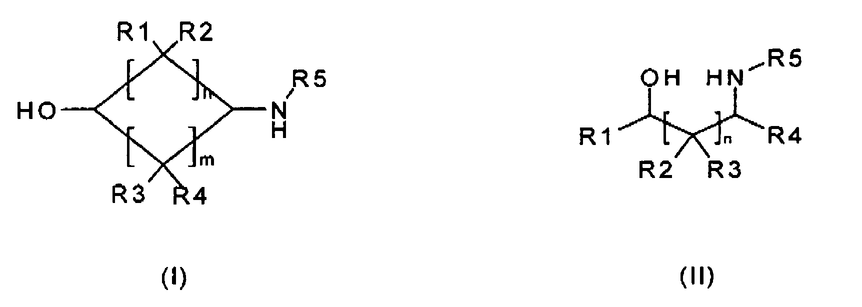 worin
unabhängig voneinander n = 0, 1, 2, 3, 4 und m = , 1, 2, 3, 4 bedeuten und
R1, R2, R3, R4 bzw. R5 unabhängig voneinander H, substituierte und unsubstituiert (C1-C8)-Alkyl, (C3-C8)-Cycloalkyl, (C1-C8)-Alkyl-(C3-C8)-Cycloalkyl, (C3-C8)-Cycloalkyl-((C1-C8)-Alkyl)1-3, (C2-C8)-Alkenyl, (C2-C8)-Alkinyl, (C6-C18)-Aryl, (C7-C19)-Aralkylrest, (C6-C18)-Aryl-((C1-C8)-Alkyl)1-3, (C3-C18)-Heteroarylrest, (C4-C19)-Heteroaralkyl, (C3-C18)-Heteroarylrest-((C1-C8)-Alkyl)1-3, bedeuten

6. Verfahren nach Anspruch 5, **dadurch gekennzeichnet, dass** in Formel (I) die Substituenten die Bedeutung R3, R4, R5 = H, n=0, m=4 haben.

7. Verfahren nach Anspruch, **dadurch gekennzeichnet, dass** als als Alkalihydroxid Kaliumhydroxid verwendet wird.

## Claims

1. A process for preparing O-alkylated amino alcohols or O-benzylated amino alcohols by reacting N-unsubstituted or N-monosubstituted amino alkoxide salts with alkyl halides or benzyl halides, the amino alkoxide salts being formed by means of alkali metal or alkaline earth metal hydroxides.

2. The process according to claim 1, wherein the reaction is carried out in a solvent.

3. The process according to claim 1, wherein is carried out in an organic solvent.

4. The process according to claim 1, wherein xylene is used as the solvent.

5. The process according to claim 1, wherein the amino alcohol has the formula (I) or (II) 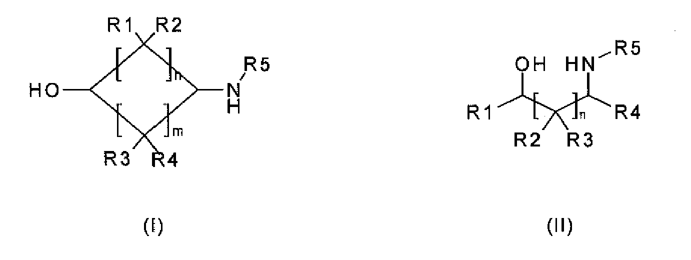 where
each independently, n = 0, 1, 2, 3, 4 and m = , 1, 2, 3, 4, and
R1, R2, R3, R4 and R5 are each independently H, substituted and unsubstituted (C₁-C₈)-alkyl, (C₃-C₈)-cycloalkyl, (C₁-C₈) -alkyl- (C₃-C₈) -cycloalkyl, (C₃-C₈) -cycloalkyl-((C₁-C₈)-alkyl)1-3, (C₂-C₈)-alkenyl, (C₂-C₈)-alkynyl, (C₆-C₁₈)-aryl, (C₇-C₁₉)-aralkyl radical, (C₆-C₁₈) -aryl- ((C₁-C₈) -alkyl) 1-3, (C₃-C₁₈) -heteroaryl radical, (C₄-C₁₉)-heteroaralkyl, (C₃-C₁₈) -heteroaryl radical ((C₁-C₈) -alkyl) 1-3.

6. The process according to claim 5, wherein the substituents in formula (I) have the definition R3, R4, R5 = H, n=0, m=4.

7. The process according to claim, wherein the alkali metal hydroxide used is potassium hydroxide.

## Revendications

1. Procédé de fabrication d'aminoalcools O-alkylés ou d'aminoalcools O-benzylés par mise en réaction de sels d'aminoalcoolates N-non substitués ou N-monosubstitués avec des halogénures d'alkyle ou des halogénures de benzyle, les sels d'aminoalcools étant formés au moyen d'hydroxydes alcalins ou alcalino-terreux.

2. Procédé selon la revendication 1, **caractérisé en ce que** la réaction est réalisée dans un solvant.

3. Procédé selon la revendication 1, **caractérisé en ce qu'**il est réalisé dans un solvant organique.

4. Procédé selon la revendication 1, **caractérisé en ce que** le xylène est utilisé en tant que solvant.

5. Procédé selon la revendication 1, **caractérisé en ce que** l'aminoalcool présente la formule (I) ou (II) 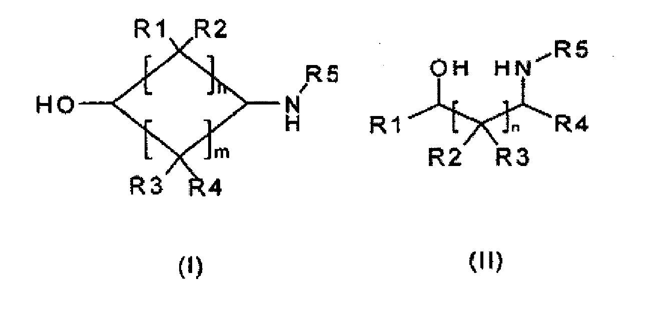 dans lesquelles
indépendamment les uns des autres, n = 0, 1, 2, 3, 4 et m = ,1, 2, 3, 4, et R1, R2, R3, R4 et R5 signifient indépendamment les uns des autres H, alkyle en (C1-C8), cycloalkyle en (C3-C8), alkyle en (C1-C8)-cycloalkyle en (C3-C8), cycloalkyle en (C3-C8-(alkyle en (C1-C8))1-3, alcényle en (C2-C8), alcynyle en (C2-C8), aryle en (C6-C18), radical aralkyle en (C7-C19), aryle en (C6-C18)-(alkyle en (C1-C8))1-3, radical hétéroaryle en (C3-C18), hétéroaralkyle en (C4-C19), radical hétéroaryle en (C3-C18)-(alkyle en (C1-C8))1-3 substitués et non substitués.

6. Procédé selon la revendication 5, **caractérisé en ce que** les substituants dans la formule (I) ont la signification R3, R4, R5 = H, n = 0, m = 4.

7. Procédé selon la revendication, **caractérisé en ce que** l'hydroxyde de potassium est utilisé en tant qu'hydroxyde alcalin.
